# EUROPEAN PATENT APPLICATION

(11) **EP 3 006 557 A1**
(43) Date of publication of application: **13.04.2016**
(21) Application number: 14188006.2
(22) Date of filing: 07.10.2014
(51) Int. Cl.: C12N 5/071, A61K 31/575

(54) **BILE ACIDS FOR INDUCING HEPATIC DIFFERENTIATION**

(71) Applicant: Heinrich Heine Universität Düsseldorf, 40225 Düsseldorf (DE)
(72) Inventor: HÄUSSINGER, Dieter, 40597 Düsseldorf (DE); KORDES, Claus, 41517 Grevenbroich (DE); SAWITZA, Iris, 50389 Wesseling (DE)
(74) Representative: Krauss, Jan

(57) **Abstract**

The present invention pertains to a method for promoting the differentiation of mesenchymal stem cells into mature hepatocytes or hepatocyte progenitors. To this end the invention employs bile acid receptor agonists, in particular bile acids such as tauroursodeoxycholic acid (TUDCA), for inducing the hepatic differentiation process in a mesenchymal stem cell population. Hepatocytes or their progenitors as produced in accordance with the methods of the invention will find application for liver regeneration, in organ or cell transplantation, and as a treatment of degenerative liver diseases such as liver cancer or liver cirrhosis.

SEQUENCE LISTING

<110>  Heinrich Heine Universitaet Duesseldorf

<120>  BILE ACIDS FOR INDUCING HEPATIC DIFFERENTIATION

<130>  U30595EP

<160>  20

<170>  PatentIn version 3.5

<210>  1
<211>  20
<212>  DNA
<213>  Mus musculus

<400>  1
acctgacagg gaagatggtg                                                   20


<210>  2
<211>  20
<212>  DNA
<213>  Mus musculus

<400>  2
gcagtggttg ataccggagt                                                   20


<210>  3
<211>  20
<212>  DNA
<213>  Mus musculus

<400>  3
cttcaaagcc tgggcagtag                                                   20


<210>  4
<211>  20
<212>  DNA
<213>  Mus musculus

<400>  4
gcactggctt atcacagcaa                                                   20


<210>  5
<211>  20
<212>  DNA
<213>  Mus musculus

<400>  5
caccattcct gcaacctttt                                                   20


<210>  6
<211>  20
<212>  DNA
<213>  Mus musculus

<400>  6

gtaccggcag gtcattcagt                                                    20

<210>  7
<211>  20
<212>  DNA
<213>  Mus musculus

<400>  7
agcctgggtc agagacagaa                                                    20

<210>  8
<211>  20
<212>  DNA
<213>  Mus musculus

<400>  8
tatctcctgc tcccacatcc                                                    20

<210>  9
<211>  21
<212>  DNA
<213>  Mus musculus

<400>  9
tgcatactgc acttcaggac a                                                  21

<210>  10
<211>  21
<212>  DNA
<213>  Mus musculus

<400>  10
ggaacaagga ctcccccttt a                                                  21

<210>  11
<211>  20
<212>  DNA
<213>  Mus musculus

<400>  11
ctgtaccctg ccatccaagt                                                    20

<210>  12
<211>  20
<212>  DNA
<213>  Mus musculus

<400>  12
tcatggattt ttggtgagca                                                    20

<210>  13
<211>  20
<212>  DNA
<213>  Mus musculus

<400>  13

aaatgtgcag gtgttgacca                                                    20

<210>  14
<211>  20
<212>  DNA
<213>  Mus musculus

<400>  14
cacgctcctc ctgaagaatc                                                    20

<210>  15
<211>  21
<212>  DNA
<213>  Mus musculus

<400>  15
aagtgttgga tacaggccag a                                                  21

<210>  16
<211>  21
<212>  DNA
<213>  Mus musculus

<400>  16
ggctttgtac ttggcttttc c                                                  21

<210>  17
<211>  20
<212>  DNA
<213>  Mus musculus

<400>  17
agtaacgtgc gtgctgacac                                                    20

<210>  18
<211>  20
<212>  DNA
<213>  Mus musculus

<400>  18
agtcgcactg gtagcaaggt                                                    20

<210>  19
<211>  20
<212>  DNA
<213>  Mus musculus

<400>  19
tggaagttta tggcctcctg                                                    20

<210>  20
<211>  20
<212>  DNA
<213>  Mus musculus

<400>  20

ccaacacagc aagaagagca 20

## Description

### FIELD OF THE INVENTION

The present invention pertains to a method for promoting the differentiation of mesenchymal stem cells into mature hepatocytes or hepatocyte progenitors. To this end the invention employs bile acid receptor agonists, in particular bile acids such as tauroursodeoxycholic acid (TUDCA), for inducing the hepatic differentiation process in a mesenchymal stem cell population. Hepatocytes or their progenitors as produced in accordance with the methods of the invention will find application for liver regeneration, in organ or cell transplantation, and as a treatment of degenerative liver diseases such as liver cancer or liver cirrhosis.

### DESCRIPTION

Hepatic failure is a terminal picture of many liver diseases such as hepatocellular carcinoma (HCC) and liver cirrhosis, and is treated by liver transplantation, but a lack of donor organs is a worldwide problem. Living donor partial liver transplantation is recognized as a measure for overcoming the lack of organs, and facilities for partial liver transplantation are increasing in the United States and European countries having advanced transplantation technology. However, partial liver transplantation cannot be considered a safe operation for adults representing the majority of transplantation patients because the resectable liver weight of donors is often less than the necessary liver weight for recipients. Thus, there is a demand for a means for safe and rapid liver regeneration for small grafts. One possibility would be the generation of a patient's own liver tissue using isolated stem cells.

In regenerative medicine, to identify a source of stem cells of high safety and efficacy is the first step of the development of bio materials for repairing and renewing damaged and defective tissues. Human embryonic stem cells (hESCs) can remain undifferentiated, if cultured under appropriate conditions, and begin to spontaneously differentiate into various types of cells, which is a good indication that a culture of embryonic stem cells is a source for producing various types of cells. However, it is not an efficient way because to control the differentiation of embryonic stem cells is required. Mesenchymal stem cells, or MSCs, are multipotent stem cells that can differentiate into a variety of cell types. MSCs have been isolated from placenta, adipose tissue, lung, bone marrow, dental pulp, and blood, and were recently found to be resident in liver as well.

Currently various protocols for directed hepatic differentiation of stem cells have been developed in the prior art as alternatives for large scale hepatocyte production. For instance, embryonic stem cells can be grown in unlimited numbers and differentiate into functional hepatocyte-like cells in culture. Maintenance, growth and differentiation, however, are time consuming and expensive. Differentiation of cells takes up to 40 days and result in a cell population of cells with variable degrees of differentiation. Further, embryonic stem cells, for now, are not considered for cell therapy applications due to risks for teratoma formation and ethical concerns. In recent years, evidence has been provided that also adult cells from various sources (bone marrow/adipose tissue/placenta/umbilical cord) could occasionally overcome lineage borders and differentiate into hepatic lineage cells upon coordinated *in vitro* stimulation. Despite remarkable progress, the resulting cells often fail to achieve complete function sufficient for regenerative therapy and toxicology assays or other pharmacological assays, remaining only, "hepatocyte-like".

Therefore, mature hepatocytes are still frequently needed for *in vitro* toxicology or pharmaceutical assays and also for cell therapies in patients with acute and metabolic liver diseases. However, liver tissues for hepatocyte isolation are rarely available. A further drawback is that hepatocytes show limited proliferation potential and dedifferentiate over time in culture. Thus, the generation of high quality hepatocytes for toxicological studies and clinical therapies remains a challenge and methods to obtain alternative cellular resources are urgently needed.

It was discovered recently that hepatic stellate cells (HSC) are liver-resident mesenchymal stem cells (MSC). Their classification as MSC is evidenced by an MSC-related expression pattern and functional analysis. Transplanted stellate cells are able to contribute to liver regeneration through differentiation into hepatocytes as reported for MSC from the bone marrow or adipose tissue. Growth factor treatment of isolated HSC from rats can initiate their differentiation into hepatocytes also *in vitro*. These findings underline the potential of MSC from various sources to generate hepatocytes for therapeutic or industrial use. The availability of hepatocytes from MSC offers perspectives to generate patient-specific livers for organ transplantation in the future. Patient-specific hepatocytes are also useful for industrial drug design, but the techniques that are available thus far are mainly based on cost-intensive growth factor treatment.

It is therefore the object of the present invention to provide an improved method for generating hepatic cells or their progenitors. These cells will be useful for liver regenerative medicine and additionally in toxicological studies for drug design. The invention seeks to simplify access to in-vitro generated hepatic cells which can be used in the treatment of degenerative liver diseases such as cancerous diseases of the liver or liver cirrhosis.

The above problem is solved in a first aspect a method of promoting hepatic differentiation of a stem cell, comprising contacting the stem cell with an effective amount of a bile acid receptor agonist.

Alternatively, the problem of the present invention is solved by a method for producing a hepatocyte or a hepatocyte progenitor cell from a stem cell via promoting hepatic differentiation in the stem cell, the method comprising the steps of providing a stem cell and contacting the stem cell with an effective amount of a bile acid receptor agonist.

The inventors have surprisingly identified that bile acids induce the hepatic differentiation in mesenchymal stem cells. This differentiation process is induced through the receptors farnesoid X receptor (FXR) and G protein-coupled bile acid receptor 1 (TGR5).

Therefore in a preferred embodiment of the above aspects of the invention said bile acid receptor agonist is a FXR and/or TGR5 agonist. Compounds falling under the meaning of the term "bile acid receptor agonist" or the term "FXR and/or TGR5 agonist" are described herein below.

A "bile acid receptor agonist" in context of the present invention is preferably selected from the group consisting of a FXR and/or TGR5 agonist. Most preferably the bile acid receptor agonist is an agonist of a mammalian homologue of FXR and/or TGR5, preferably human FXR and/or TGR5. However, also other receptors such as human steroid and xenobiotic receptor (SXR) and its rodent homolog pregnane X receptor (PXR), vitamin D receptor (VDR), and constitutive androstane receptor (CAR) are known to respond to bile acids and shall form part of the herein described invention. Furthermore comprised are any receptors that bind and react to bile acids. Those receptors might at present lack the annotation as bile acid receptors, however, they can be easily identified using standard screening procedures, such as for example, genome wide knock-out screens, such as siRNA screens.

As used herein, the term "bile acid receptor agonist" means a substance that affects an increase in the amount or rate of bile acid receptor expression or activity. Such a substance can act directly, for example, by binding to a bile acid receptor and increasing the amount or rate of the bile acid receptor expression or activity. A bile acid receptor agonist can also increase the amount or rate of the bile acid receptor expression or activity, for example, by binding to a bile acid receptor in such a way as to enhance or promote interaction of the bile acid receptor with a bile acid receptor ligand, such as bile acid; by binding to a bile acid receptor and modifying it, such as by removal or addition of a moiety; and by binding to a bile acid receptor and enhancing its stability. A bile acid receptor agonist can also act indirectly, for example, by binding to a regulatory molecule or gene region so as to modulate regulatory protein or gene region function and affect an increase in the amount or rate of a bile acid receptor expression or activity. Thus, a bile acid receptor agonist can act by any mechanisms that result in increase in the amount or rate of bile acid receptor expression or activity. As mentioned before, a preferred bile acid receptor of the invention is FXR and/or TGR5.

A bile acid receptor agonist can be, for example, a naturally or non-naturally occurring macromolecule, such as a polypeptide, peptide, peptidomimetic, nucleic acid, carbohydrate or lipid. A bile acid receptor agonist further can be an antibody, or antigen-binding fragment thereof, such as a mono-clonal antibody, humanized antibody, chimeric antibody, minibody, bifunctional antibody, single chain antibody (scFv), variable region fragment (Fv or Fd), Fab or F(ab)2. A bile acid receptor agonist can also be a polyclonal antibody specific for a bile acid receptor. A bile acid receptor agonist further can be a partially or completely synthetic derivative, analog or mimetic of a naturally occurring macromolecule, or a small organic or inorganic molecule.

A bile acid receptor agonist that is an antibody can be, for example, an antibody that binds to a bile acid receptor and activates receptor signalling by mimicking ligand binding, or alters the activity of a molecule that regulates bile acid receptor expression or activity, such that the amount or rate of bile acid receptor expression or activity is increased. An antibody useful in a method of the invention can be a naturally occurring antibody, including monoclonal or polyclonal antibodies or fragments thereof, or a non-naturally occurring antibody, including but not limited to a single chain antibody, chimeric antibody, bifunctional antibody, complementarity determining region-grafted (CDR-grafted) antibody and humanized antibody or an antigen-binding fragment thereof.

Preferably the bile acid receptor agonist of the invention is a bile acid, an analog thereof, a derivative thereof or a salt thereof.

The above methods are in preferred embodiments *ex-vivo* or *in-vitro* methods.

The term "stem cell" refers to a cell possessing two properties: (1) self-renewal and (2) potency, wherein the term "self-renewal" means the ability of the cell to go through numerous cycles of cell division while maintaining the undifferentiated state; and wherein the term "potency" means the capacity of the cell to differentiate into specialized cell types. Preferably, the stem cell of the present invention is pluripotent, multipotent, or unipotent stem cell. More preferably, the stem cell of this invention is pluripotent stem cell. The term "pluripotent" refers to a stem cell that can differentiate into cells derived from any of the three germ layers viz. mesoderm, endoderm, and ectoderm from which all the cells of the body arise. The term "multipotent" refers to a stem cell that can produce only cells of a closely related family of cells. The term "unipotent" refers to a stem cell that can differentiate into only one cell type.

Preferably the stem cell of the invention is a stem cell having the ability to differentiate into a hepatocyte progenitor cell or a mature hepatocyte. Thus, most preferably the stem cell of the present invention is an embryonic stem cell or a pluripotent adult stem cell. An embryonic stem cell according to the present invention is obtainable by a method that does not involve the destruction of human embryos. More preferably the stem cell of the present invention is a mesenchymal stem cell (MSC).

As used herein, the term "mesenchymal stem cells" includes somatic stem cells derived from mesenchyme. These mesenchymal stem cells can differentiate into mesenchymal cells. Examples of the "mesenchymal stem cells" include liver resident MSC, bone marrow mesenchymal stem cells and umbilical cord blood-derived stem cells. The mesenchymal stem cells seem to be present in all tissues containing a mesenchymal tissue. Among the mesenchymal tissues, the bone marrow mesenchymal stem cells, however, can be easily collected by bone marrow aspiration, and their culture technique has been established.

A preferred MSC of the invention is a liver resident MSC (hepatic stellate cell (HSC)), or a bone marrow MSC.

The terms "proliferation" and "expansion" as used interchangeably herein refer to an increase in the number of cells of the same type by division. The term "differentiation" refers to a process whereby cells become specialized for a particular function, for example, where cells acquire one or more morphological or genetic characteristics and/or functions different from that of the initial cell type. The term includes both lineage commitment and terminal differentiation processes. Differentiation may be assessed, for example, by monitoring the presence or absence of differentiation markers, using immuno-histochemistry, gene expression assays, or other procedures known to a skilled in the art. Differentiated progeny cells derived from progenitor cells may be, but are not necessarily, related to the same germ layer or tissue as the source tissue of the stem cells. Thus, the term "hepatic differentiation" means in the present context the process of specialization of a cell to develop into a hepatic progenitor cell (immature hepatocyte) or into a mature hepatocyte.

The term "hepatocyte" described herein means a cell having ability to produce protein such as albumin and various blood coagulation factors which are index of liver function, ability of gluconeogenesis, ability to produce carbamide, abilities of detoxication and purification of blood, and ability to metabolize amino acid, glucide and lipid. Examples thereof include hepatocyte, liver sinusoid endothelial cell, liver stellate cell, liver Pit cell, Kupffer cell and the like.

The term "bile acid" in accordance with the present invention embraces all bile acids and their salts, as well as analogs and derivatives thereof. Bile acids are compounds including, but not limited to, a steroid acid, or salt thereof, including cholic acid, taurocholic acid, glycocholic acid, lithocholic acid, chenodeoxycholic acid, deoxycholic acid, glycodeoxycholic acid, derivatives thereof, and mixtures thereof. The term is intended to encompass any such compound recognized by a person of skill in the art as a bile-acid or a cholate derivative. The term "bile acid salt" includes mixtures of bile acid salts. Exemplary bile acid salts include the salts of dihydroxy cholic acids, such as deoxycholic acid, glycodeoxycholic acid, taurodeoxycholic acid, chenodeoxycholic acid, glycochenodeoxycholic acid, and taurocheno deoxycholic acid, and trihydroxy cholic acids, such as cholic acid, glycocholic acid, and taurocholic acid. The acid addition salts include sodium, and potassium salts.

Preferred bile acids of the invention are selected from the group consisting of tauroursodeoxycholic acid (TUDCA), cholic acid (CA), gly-cochenodeoxycholic acid (GCDCA), taurocholic acid (TCA), taurochenodeoxycholic acid (TCDCA), and taurolithocholic acid (TLCA), and analogs and derivatives of these compounds. Most preferred is TUDCA as bile acid of the invention.

Examples of known derivatives of natural bile acids are for example described in European Patent No. 0 312 867, which describes 6-methyl derivatives of natural biliary acids such as ursodeoxycholic acid, ursocholic acid, chenodeoxycholic acid and cholic acid. Other derivatives are for example disclosed in US 2014/024631which are however only exemplary possibilities of derivatization of bile acids.

In one embodiment of the invention hepatic differentiation is characterized by an increased expression of at least one hepatocyte differentiation marker upon contacting the stem cells with the bile acid receptor agonist. A hepatocyte differentiation marker is preferably selected from the group consisting of keratin 19 (K19), epithelial cell adhesion molecule (EPCAM), α-fetoprotein (AFP), albumin, keratin 18, cytochrome P450 7A1 (CYP7A1) and hepatocyte nuclear factor 4α (HNF4α), and preferably is albumin, most preferably albumin released into the culture supernatant.

In this context the increased expression of a hepatocyte differentiation marker selected from keratin 19 (K19), epithelial cell adhesion molecule (EPCAM) and α-fetoprotein (AFP), is indicative for a hepatocyte progenitor (an immature hepatocyte); whereas the increased expression of a hepatocyte differentiation marker selected from albumin, keratin 18, cytochrome P450 7A1 (CYP7A1) and hepatocyte nuclear factor 4α (HNF4α) is indicative for a (mature) hepatocyte.

In order to test whether the stem cells after undergoing the procedures of the invention have sufficiently differentiated into hepatocyte progenitors or hepatocytes, an optional further step of testing the expression of at least one hepatocyte differentiation marker as mentioned above is included. Preferably the testing is performed after the differentiation process is completed. More preferably the expression of one or more of the hepatic differentiation markers is monitored throughout the procedure to observe the degree of maturity of the cells used in the methods of the invention. Thereby the invention allows differentiating the stem cells to a specifically desired stage of hepatic differentiation.

For example, the expression of at least one hepatocyte differentiation marker may be tested by protein detection, using immuno-histochemical procedures such as ELISA, or mass spectrometry. Alternatively the mRNA expression of the hepatic differentiation markers of the invention may be assayed, for example by quantitative real time PCR (qPCR). Methods for assaying biomarker expression are well-known to the person of skill the art.

In one preferred embodiment of the methods of the present invention, the effective amount of bile acid is a concentration of 0.1 µM to 500µM, preferably 0.5µM to 100µM, 1µM to 50µM, 1µM to 5µM, or most preferably 1µM to 3µM, such as about 2µM. The term "about" shall mean a deviation of the respective numerical value of +/- 20% or less. These amounts are particularly preferred if TUDCA is selected as the preferred bile acid of the present invention.

The "contacting" of the bile acid receptor agonist with the stem cell in accordance with the herein disclosed invention is preferably a culturing of the stem cells in the presence of the bile acid receptor agonist. The culturing in one preferred embodiment is a culturing in serum-free medium supplemented with the bile acid receptor agonist.

Yet a further embodiment relates to the methods of the invention, where the contacting takes place for at least 1 day, preferably 5 days, or even more preferably at least 10, 15, 20, 30, 40 or more days. Depending on how long the stem cells are contacted with the bile acid receptor agonists of the invention, the degree of hepatic differentiation increases, meaning that the longer the cells are in contact with the bile acid receptor agonist the more mature the hepatic cells get. Preferably a contacting for at least 5 days already induces the expression of hepatic differentiation markers (such as albumin) and therefore is already sufficient to generate at least a hepatic progenitor cell.

The problem of the invention is solved in a further aspect by bile acid receptor agonist (as defined herein above and below) for use in the treatment of a patient suffering from a liver disease.

Alternatively provided is a bile acid receptor agonist (as defined herein above and below) for use in the treatment of a patient suffering from a liver disease, wherein the treatment is a cell, tissue or organ transplantation and comprises the steps of a method for producing a hepatocyte progenitor cell or a hepatocyte of the invention, or a method for inducing hepatic differentiation as described herein before, and administering said hepatocyte or hepatocyte progenitor cell to the patient.

A preferred embodiment of this aspects relates to a bile acid receptor agonist, which is a farnesoid X receptor (FXR) agonist and/or a G protein-coupled bile acid receptor 1 (TGR5) agonist, such as a bile acid selected from the group comprising tauroursodeoxycholic acid (TUDCA), cholic acid (CA), glycochenodeoxycholic acid (GCDCA), taurocholic acid (TCA), taurochenodeoxycholic acid (TCDCA), and taurolithocholic acid (TLCA), and preferably is TUDCA.

Yet another embodiment pertains to the above described medical use, wherein said stem cell is an autologous stem cell from said patient, and optionally comprises as first step obtaining the autologous stem cell from the patient.

In this context the administration of the cells is a transplantation, for example a transplantation of the hepatocyte or hepatocyte progenitor cell into the liver of the patient. A patient in context of the herein described invention is preferably a mammal, for example a mammal suffering from a degenerative liver disease, most preferably a human patient.

In the context of the herein described invention the term "liver disease" may pertain to hepatitis, cirrhosis, acute liver failure, acute liver infections, acute chemical toxicity, chronic liver failure, cholangiocitis, biliary cirrhosis, Alagille syndrome, alpha-1-antitrypsin deficiency, autoimmune hepatitis, biliary atresia, cancer of the liver, cystic disease of the liver, fatty liver, galactosemia, gallstones, Gilbert's syndrome, hemochromatosis, hepatitis A, hepatitis B, hepatitis C and other hepatitis viral infections, poryphyria, primary sclerosing cholangitis, Reye's syndrome, sarcoidosis, tyrosinemia, type 1 glycogen storage disease or Wilson's disease. Another embodiment then relates to the medical use of the bile acid receptor agonist of the invention, wherein a treatment comprises the administration of the bile acid receptor agonist to the patient.

The object of the present invention is furthermore solved by the use of a bile acid receptor agonist (as described herein before) for inducing hepatocyte differentiation of a stem cell. The use according to the invention is preferably an *in-vitro* or *ex-vivo* use.

Also provided is a kit adapted for the performance of a method according to any of the herein described aspects and embodiments of the invention.

Another aspect of the invention further relates to a method for identifying (screening) components of the herein described bile acid dependent hepatic differentiation. The method comprises the steps of providing a stem cell wherein a candidate gene is either overexpressed or inhibited, contacting this stem cell with a bile acid agonist of the invention, and observing whether said stem cell increases the expression of at least one hepatic differentiation marker as described herein before. In case the stem cell does not increase the expression of said hepatic differentiation marker, the candidate gene is necessary for bile acid dependent hepatic differentiation. A preferred hepatic differentiation marker can be albumin. Alternatively reporter assays can be used. This assay can in particular be used to screen for novel bile acid receptors.
Further provided is a method for liver tissue engineering, the method comprising the steps of providing a decellularized liver, or decellularized parts of a liver (liver-tissue), contacting said decellularized liver, or decellularized parts of a liver, with stem cells (as described herein before) under conditions in which said stem cells engraft, multiply or differentiate within said decellularized liver, or decellularized parts of a liver, to obtain a recellularized liver, or recellularized parts of a liver, and contacting said recellularized liver, or recellularized parts of a liver, with a bile acid receptor agonist as defined herein before. In accordance with the present invention contacting the recellularized tissue with a bile acid receptor agonist (preferably a bile acid or a derivative or analog thereof) induces hepatocyte differentiation and therefore allows to regenerate liver tissue, or even a complete liver.

The bile acid receptor agonist used in the tissue engineering method is preferably as described herein before.

Preferably said decellularized liver, or decellularized parts of the liver comprise a decellularized extracellular matrix of a liver or parts thereof, wherein said extracellular matrix comprises an exterior surface, and wherein said extracellular matrix, including the vasculature bed, substantially retains the morphology of said extracellular matrix prior to decellularization, and wherein said exterior surface is substantially intact.

In another embodiment the decellularized liver or decellularized parts of a liver are cannulated in order to allow the perfusion of said decellularized liver or decellularized parts of a liver with stem cells, and thereafter to perfuse with the bile acid receptor supplemented medium in order to induce hepatic differentiation.

How to decellularize organs such as a liver is known in the art, for example such methods are disclosed in WO 2007/025233 or in Wang X et al., 2014, J Biomed Mater Res A. 2014 Apr;102(4):1017-25. doi: 10.1002/jbm.a.34764 (both documents are incorporated by reference in its entirety).

Stems cells as defined herein above can be introduced ("seeded") into a decellularized liver or liver-tissue by injection into one or more locations. In addition, more than one type of cell (i.e., a cocktail of cells) can be introduced into a decellularized liver or liver-tissue. For example, a cocktail of cells can be injected at multiple positions in a decellularized liver or liver-tissue or different cell types can be injected into different portions of a decellularized liver or tissue. Alternatively, or in addition to injection, stem cells or a cocktail of stem cells can be introduced by perfusion into a cannulated decellularized liver or liver-tissue. Method for cannulization of organs are well known to the person of skill. For example, stem cells can be perfused into a decellularized liver using a perfusion medium, which can then be changed to an expansion and/or differentiation medium (in particular a medium supplemented with a bile acid receptor agonist) to induce growth and/or differentiation of the stem cells in accordance with the present invention. During recellularization, a liver or liver tissue is maintained under conditions in which at least some of the stem cells can multiply and/or differentiate within and on the decellularized organ or tissue. Those conditions include, without limitation, the appropriate temperature and/or pressure, electrical and/or mechanical activity, force, the appropriate amounts of 02 and/or CO2, an appropriate amount of humidity, and sterile or near-sterile conditions. During recellularization, the decellularized organ or tissue and the stem cells attached thereto are maintained in a suitable environment. For example, the stem cells may require a nutritional supplement (e.g., nutrients and/or a carbon source such as glucose), exogenous hormones or growth factors, and/or a particular pH.

In preferred embodiments the recellularized liver according to the invention is to be transplanted into a patient in need of liver-tissue transplantation. In those cases, the stem cells used to recellularize a decellularized organ or tissue can be obtained from the patient such that the stem cells are "autologous" to the patient. Autologous adult stem cells may be obtained from a patient using methods known to the skilled artisan. However, it is also possible to generate a transplant organ from cells of a donor, preferably wherein said donor is a close relative to the patient such as a sibling. Irrespective of the source of the stem cells (e.g., autologous or not), the decellularized liver can be autologous, allogeneic or xenogeneic to a patient. The recellularization may be performed both *in-vitro* or *in-vivo.*

In this regard, the invention additionally relates to an artificial liver or artificial liver-tissue produced according to the above method for liver tissue engineering.

Another aspect then pertains to a use of a bile acid receptor agonist of the invention in tissue engineering. Preferably the tissue engineering comprises the use of a decellularized liver or decellularized parts of a liver.

Another aspect of the present application relates to compositions and kits for treating or preventing a liver disease using the compounds or combinations of the invention. In one embodiment, the composition comprises compounds as described herein above, wherein the compounds are preferably selected from an antibody, antibody fragment, agent-encoding expression vector, small molecules, and bile acids together with a pharmaceutically acceptable carrier.

As used herein the language "pharmaceutically acceptable carrier" is intended to include any and all solvents, solubilizers, fillers, stabilizers, binders, absorbents, bases, buffering agents, lubricants, controlled release vehicles, diluents, emulsifying agents, humectants, lubricants, dispersion media, coatings, antibacterial or antifungal agents, isotonic and absorption delaying agents, and the like, compatible with pharmaceutical administration. The use of such media and agents for pharmaceutically active substances is well-known in the art. Except insofar as any conventional media or agent is incompatible with the active compound, use thereof in the compositions is contemplated. Supplementary agents can also be incorporated into the compositions. In certain embodiments, the pharmaceutically acceptable carrier comprises serum albumin.

The pharmaceutical composition of the invention is formulated to be compatible with its intended route of administration. Examples of routes of administration include parenteral, e.g., intrathecal, intra-arterial, intravenous, intradermal, subcutaneous, oral, transdermal (topical) and transmucosal administration.

Solutions or suspensions used for parenteral, intradermal, or subcutaneous application can include the following components: a sterile diluent such as water for injection, saline solution, fixed oils, polyethylene glycols, glycerine; propylene glycol or other synthetic solvents; antibacterial agents such as benzyl alcohol or methyl parabens; antioxidants such as ascorbic acid or sodium bisulfate; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL™ (BASF, Parsippany, N.J.) or phosphate buffered saline (PBS). In all cases, the injectable composition should be sterile and should be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyetheylene glycol, and the like), and suitable mixtures thereof. The proper fluidity can be maintained, for example, by the use of a coating such as lecithin, by the maintenance of the requited particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as manitol, sorbitol, and sodium chloride in the composition. Prolonged absorption of the injectable compositions can be brought about by including in the composition an agent which delays absorption, for example, aluminum monostearate and gelatin.

Sterile injectable solutions can be prepared by incorporating the active compound (e.g., a neuregulin) in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

Oral compositions generally include an inert diluent or an edible carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Stertes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from pressured container or dispenser which contains a suitable propellant, e.g., a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the pharmaceutical compositions are formulated into ointments, salves, gels, or creams as generally known in the art.

In certain embodiments, the pharmaceutical composition is formulated for sustained or controlled release of the active ingredient. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from e.g. Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art.

It is especially advantageous to formulate oral or parenteral compositions in dosage unit form for ease of administration and uniformity of dosage. Dosage unit form as used herein includes physically discrete units suited as unitary dosages for the subject to be treated; each unit containing a predetermined quantity of active compound calculated to produce the desired therapeutic effect in association with the required pharmaceutical carrier. The specification for the dosage unit forms of the invention are dictated by and directly dependent on the unique characteristics of the active compound and the particular therapeutic effect to be achieved, and the limitations inherent in the art of compounding such an active compound for the treatment of individuals.

Toxicity and therapeutic efficacy of such compounds can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compounds which exhibit large therapeutic indices are preferred. While compounds that exhibit toxic side effects may be used, care should be taken to design a delivery system that targets such compounds to the site of affected tissue in order to minimize potential damage to uninfected cells and, thereby, reduce side effects.

The data obtained from the cell culture assays and animal studies can be used in formulating a range of dosage for use in humans. The dosage of such compounds lies preferably within a range of circulating concentrations that include the ED50 with little or no toxicity. The dosage may vary within this range depending upon the dosage form employed and the route of administration utilized. For any compound used in the method of the invention, the therapeutically effective dose can be estimated initially from cell culture assays. A dose may be formulated in animal models to achieve a circulating plasma concentration range that includes the IC50 (i.e., the concentration of the test compound which achieves a half-maximal inhibition of symptoms) as determined in cell culture. Such information can be used to more accurately determine useful doses in humans. The pharmaceutical compositions can be included in a container, pack, or dispenser together with instructions for administration.

The present invention will now be further described in the following examples with reference to the accompanying figures and sequences, nevertheless, without being limited thereto. For the purposes of the present invention, all references as cited herein are incorporated by reference in their entireties. In the Figures:

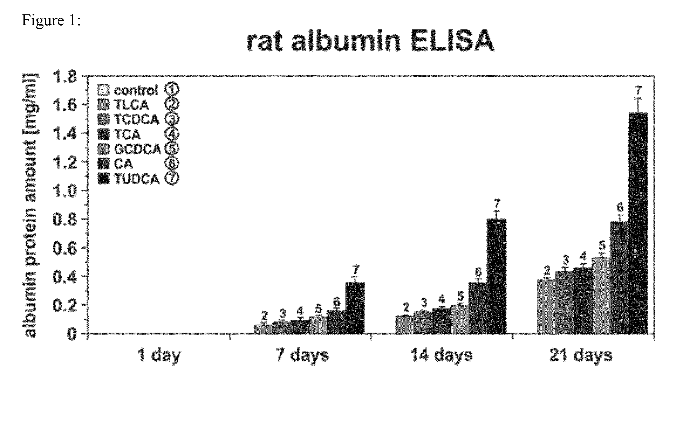

**Figure 1****:** HSC of rats differentiate into hepatocytes in response to bile acid treatment in primary culture. Primary cultures of rat HSC were treated with 2 µM of either GCDCA (glycochenodeoxycholic acid), TUDCA (tauroursodeoxycholic acid), CA (cholic acid), GCDCA (glycochenodeoxycholic acid), TCA (taurocholic acid), TCDCA (taurochenodeoxycholic acid), or TLCA (taurolithocholic acid) for 21 days using serum-free culture medium. The albumin concentration of the culture supernatants was determined by an ELISA specific for rat albumin in three independent experiments [n = 3]. The induction of albumin production indicated the differentiation of stellate cells into hepatocytes in response to bile acid treatment.

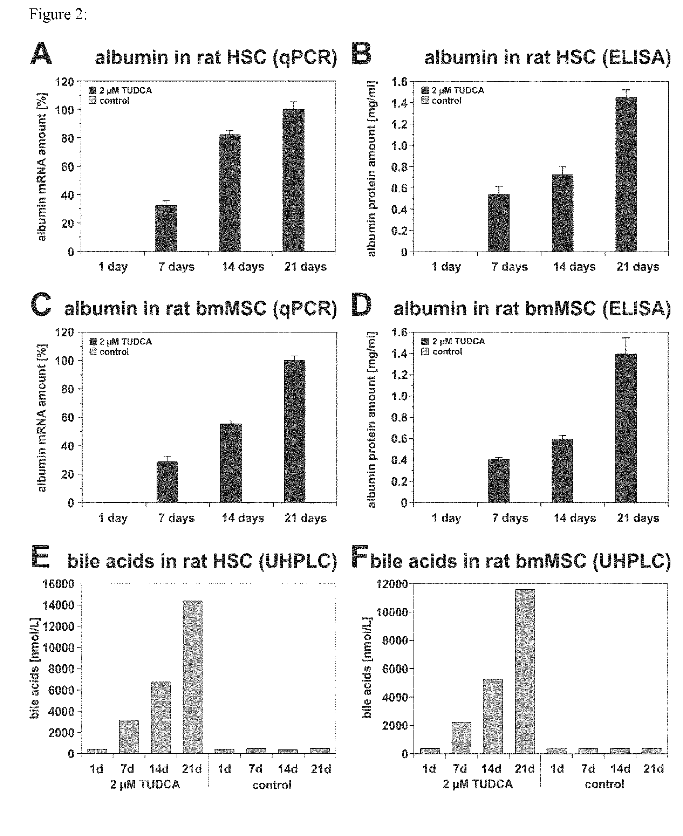

**Figure 2****:** TUDCA promotes hepatic differentiation of HSC and bmMSC of rats. HSC and bmMSC from rats were treated with 2 µM of TUDCA (tauroursodeoxycholic acid) for 21 days using serum-free culture medium. The mRNA of albumin was determined by qPCR in adherent cells. The albumin and bile acid concentration of the culture supernatants was determined by an ELISA specific for rat albumin and UHPLC-MS/MS in three independent experiments [n = 3]. The concentrations of bile acids measured by UHPLC-MS/MS (Figure 2E and F) are provided in Table 2 and 3. The induction of albumin expression and bile acid production indicated the differentiation of HSC and bmMSC into hepatocytes in response to TUDCA treatment.

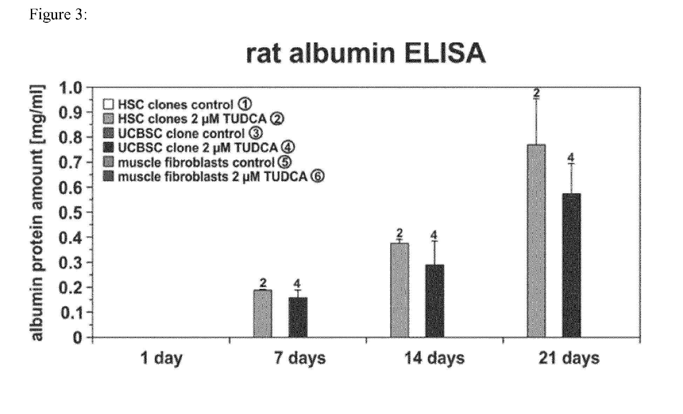

**Figure 3****:** Clonally expanded HSC and MSC from the umbilical cord blood of rats differentiate into hepatocytes by TUDCA treatment. Cell clones of HSC and umbilical cord blood stem cells (UCBSC) as well as muscle fibroblasts from rats were treated with 2 µM of TUDCA (tauroursodeoxycholic acid) for 21 days using serum-free culture medium. The albumin concentration of the culture supernatants was determined by an ELISA specific for rat albumin in three independent experiments [n = 3]. The induction of albumin expression indicated the differentiation of HSC clones and UCBSC into hepatocytes in response to TUDCA treatment. No albumin synthesis was found in cell clones of HSC and UCBSC without TUDCA treatment (control). Neither TUDCA-treated nor untreated (control) muscle fibroblasts expressed albumin. This indicates that muscle fibroblasts were unable to differentiate into hepatocytes.

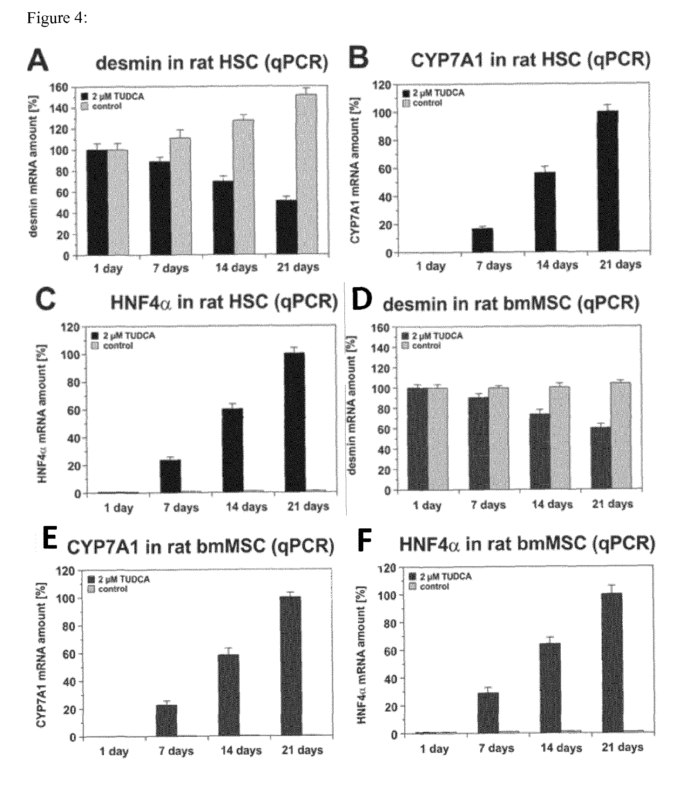

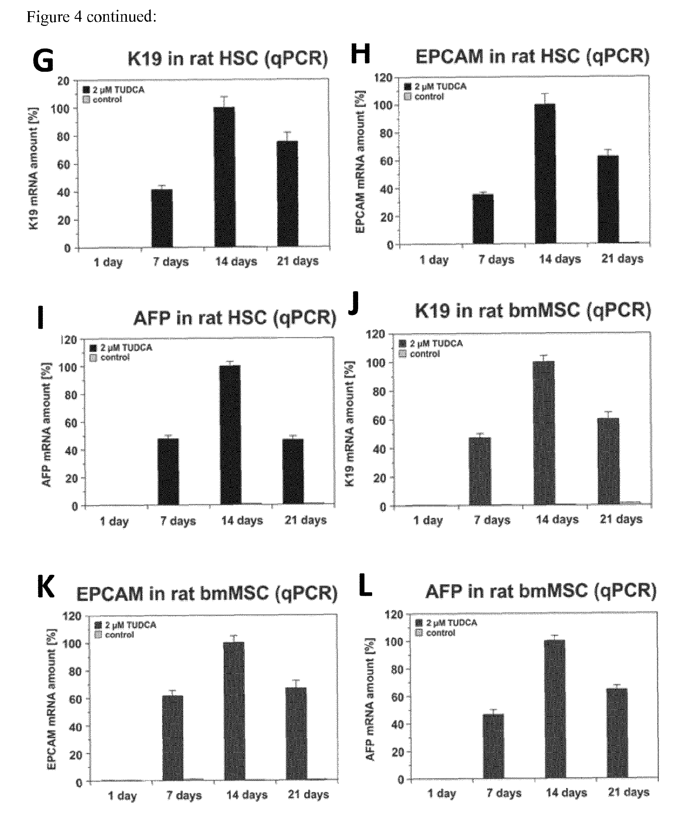

**Figure 4****:** Intermediate states of mesenchymal and epithelial cells appear in TUDCA-treated HSC and bmMSC of rats during hepatic differentiation as investigated by qPCR. HSC and bmMSC from rats were treated with 2 µM of TUDCA (tauroursodeoxycholic acid) for 21 days using serum-free culture medium. The expression of mesenchymal cells (desmin), progenitor cells (K19, EPCAM, AFP) and hepatocytes (CYP7A1, HNF4α) during TUDCA treatment was analyzed by qPCR in three independent experiments [n = 3]. The transient appearance of K19, EPCAM and AFP indicated the formation of hepatic progenitor cells during differentiation of HSC and bmMSC into hepatocytes in response to TUDCA treatment. The expression of CYP7A1 and HNF4α indicated increased hepatocyte formation with time.

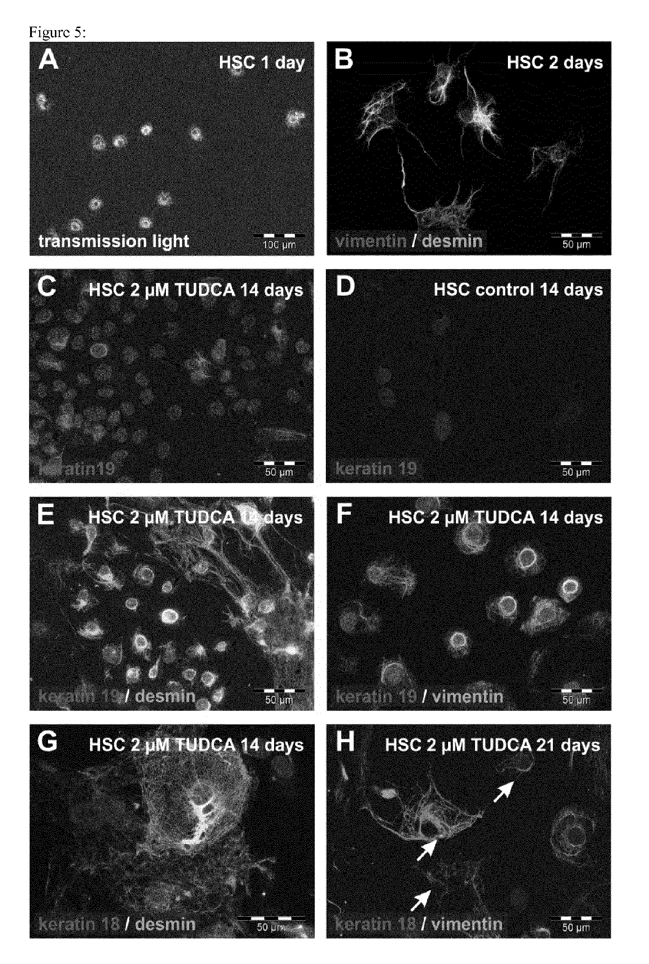

**Figure 5****:** TUDCA induces intermediate states of mesenchymal and epithelial cells in primary cultures of rat HSC during hepatic differentiation. Rat HSC were treated with 2 µM of TUDCA (tauroursodeoxycholic acid) for 21 days using serum-free culture medium. The expression of mesenchymal (desmin, vimentin) and epithelial (K18, K19) markers was analyzed by immunofluorescence during TUDCA treatment. The simultaneous expression of mesenchymal and epithelial proteins supported the conclusion that progenitor cells and hepatocytes originated from HSC with mesenchymal characteristics. More mature hepatocytes (K18, arrows) were found later during TUDCA treatment (21 days).

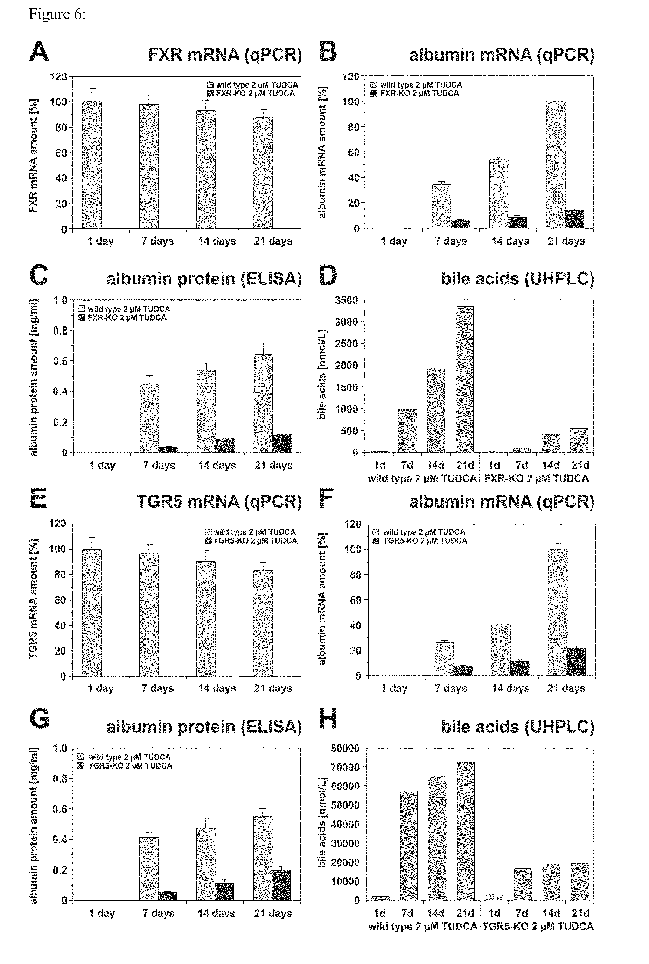

**Figure 6****:** TUDCA promotes hepatic differentiation via FXR and TGR5 in bmMSC of mice. MSC were isolated from bone marrow of FXR and TGR5 knockout (KO) mice and treated with 2 µM of TUDCA (tauroursodeoxycholic acid) for 21 days using serum-free culture medium. MSC from the bone marrow of corresponding wild type mouse strains without knockout of the bile acid receptors were used as controls. The mRNA of FXR, TGR5 and albumin was determined by qPCR in adherent cells. The albumin and bile acid concentration of the culture supernatants was determined by an ELISA specific for rat albumin and UHPLC-MS/MS in three independent experiments [n = 3]. The concentrations of bile acids measured by UHPLC-MS/MS (Figure 6D and H) are provided in Table 4 and 5. The lack of FXR and TGR5 expression confirmed the knockout of these bile acid receptors in bmMSC from FXR and TGR5 knockout mice. FXR and TGR5 expression was only found in bmMSC from corresponding wild type mice. TUDCA-mediated hepatic differentiation was significantly inhibited in bmMSC from FXR and TGR5 knockout mice as indicated by the low mRNA and protein amounts of albumin as well as inhibited bile acid production in comparison to bmMSC from wild type mice. Thus, TUDCA-induced hepatic differentiation involves FXR and TGR5.

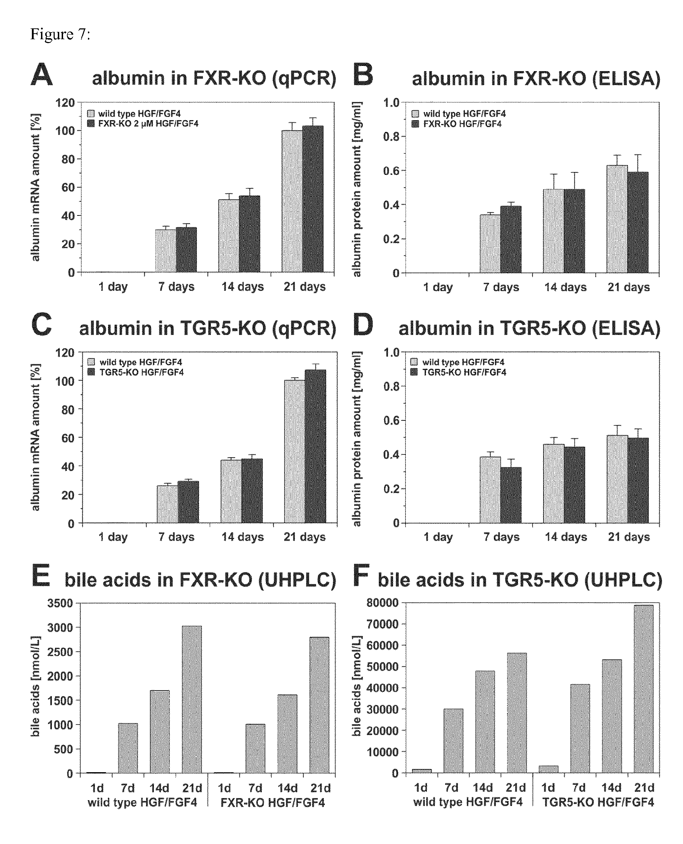

**Figure 7****:** HGF and FGF4 induce hepatic differentiation independent of FXR and TGR5 in bmMSC of mice. MSC were isolated from bone marrow of FXR and TGR5 knockout (KO) mice and treated with 40 ng/ml HGF and 50 ng/ml FGF4 for 21 days using serum-free culture medium. MSC from the bone marrow of corresponding wild type mouse strains without knockout of the bile acid receptors were used as controls. The mRNA of albumin was determined by qPCR in adherent cells. The albumin and bile acid concentration of the culture supernatants was determined by an ELISA specific for rat albumin and UHPLC-MS/MS in three independent experiments [n = 3]. The concentrations of bile acids measured by UHPLC-MS/MS (Figure 7E and F) are provided in Table 6 and 7. Growth factor-mediated hepatic differentiation was found to be independent from bile acid receptors FXR and TGR5, since the albumin expression and bile acid production remained unchanged under all experimental conditions.

**SEQ ID NO: 1 to 20:** Q-PCR Primer Sequences

### EXAMPLES

### Material and Methods

MSC were treated with 2 µM of individual bile acids, which were dissolved in a serum-free medium consisting of Dulbecco's Modified Eagle Medium (DMEM, #41966-029, Gibco, Invitrogen, Karlsruhe, Germany) supplemented with 1% (v/v) insulin-transferrin-sodium selenite (ITS, #I1884, Sigma-Aldrich, St. Louis, MO, USA), 1% linolic acid-albumin from bovine serum (#L9530, Sigma-Aldrich) and 1% antibiotic/antimycotic solution (#15240-062, Gibco). The medium was exchanged completely every second to third day and samples for the analysis of mRNA, albumin and bile acid production were taken weekly. The day 1 represents the beginning of the experiment without bile acid or growth factor treatment. Stellate cells were isolated from the liver of rats and treated with bile acids one day after isolation. MSC of the bone marrow were obtained by flushing out the bone marrow of long bones from rats and mouse.

After depletion of hematopoietic cell lineages, bmMSC were first expanded for at least 14 days in culture before the experiment was initiated. Muscle fibroblasts and clonally expanded HSC or MSC from umbilical cord blood cells were maintained in culture for several months before bile acid treatment. The bile acids were obtained from Sigma-Aldrich and dissolved in water. Mesenchymal and epithelial cell markers characteristic for hepatic progenitor cells and hepatocytes were determined by quantitative polymerase chain reaction (qPCR) and immunofluorescence staining during bile acid-mediated differentiation of MSC. Primer sets used for qPCR are provided in Table 1.

The mRNA of the gene hypoxanthine-guanine phosphoribosyltransferase 1 (HPRT1) was used for the normalization of the qPCR results. In separate experiments, bmMSC of FXR and TGR5 knockout mice were treated with 50 ng/ml human FGF4 (#100-31, PreproTech, Rocky Hill, NJ, USA) and 40 ng/ml human HGF (#100-39, PreproTech) dissolved in DMEM supplemented with 1% insulin-transferrin-sodium selenite (ITS, #I1884, Sigma-Aldrich), 1% linolic acid-albumin from bovine serum (#L9530, Sigma-Aldrich) and 1% antibiotic/antimycotic solution (Gibco) to initiate hepatic differentiation.

**Table 1: Primer sets for qPCR**

| **Gene** | **Forward Primer** | **Reverse Primer** | **bp** | **Accession No.** |
|---|---|---|---|---|
| **α-fetoprotein** | ACCTGACAGGGAAG ATGGTG (SEQ ID NO:1) | GCAGTGGTTGATA CCGGAGT(SEQ ID NO:2) | 155 | NM_012493 |
| **albumin** | CTTCAAAGCCTGGGC AGTAG(SEQ ID NO:3) | GCACTGGCTTATC ACAGCAA(SEQ ID NO:4) | 221 | V01222 |
| **CYP7A1** | CACCATTCCTGCAAC CTTTT(SEQ ID NO:5) | GTACCGGCAGGTC ATTCAGT(SEQ ID NO:6) | 170 | NM_012942 |
| **desmin** | AGCCTGGGTCAGAG ACAGAA(SEQ ID NO:7) | TATCTCCTGCTCC CACATCC(SEQ ID NO:8) | 166 | NM_022531 |
| **EPCAM** | TGCATACTGCACTTC AGGACA(SEQ ID NO:9) | GGAACAAGGACT CCCCCTTTA(SEQ ID NO:10) | 195 | NM_138541 |
| **FXR** | CTGTACCCTGCCATC CAAGT(SEQ ID NO:11) | TCATGGATTTTG GTGAGCA(SEQ ID NO:12) | 157 | AY094586 |
| **HNF4α** | AAATGTGCAGGTGTT GACCA(SEQ ID NO:13) | CACGCTCCTCCTG AAGAATC(SEQ ID NO:14) | 178 | NM_022180 |
| **HPRT1** | AAGTGTTGGATACAG GCCAGA(SEQ ID NO:15) | GGCTTTGTACTTG GCTTTTCC(SEQ ID NO:16) | 145 | NM_012583 |
| **K19** | AGTAACGTGCGTGCT GACAC(SEQ ID NO:17) | AGTCGCACTGGTA GCAAGGT(SEQ ID NO:18) | 193 | NM_199498 |
| **TGR5** | TGGAAGTTTATGGCC TCCTG(SEQ ID NO:19) | CCAACACAGCAA GAAGAGCA(SEQ ID NO:20) | 209 | NM_174985 |

**Table 2: Refers to the data in Figure 2E**

| **bile acid [nmol/L]** | **rat HSC 2 µM TUDCA** | | | | **rat HSC control** | | | |
|---|---|---|---|---|---|---|---|---|
| | **1d** | **7d** | **14d** | **21d** | **1d** | **7d** | **14d** | **21d** |
| CA | 119 | 718 | 1257 | 2810 | 119 | 181 | 75 | 157 |
| CDCA | 0 | 329 | 694 | 1394 | 0 | 0 | 0 | 0 |
| DCA | 245 | 866 | 1741 | 3406 | 245 | 228 | 213 | 235 |
| UDCA | 0 | 43 | 61 | 156 | 0 | 0 | 0 | 0 |
| HDCA | 0 | 186 | 442 | 965 | 0 | 0 | 0 | 0 |
| α/ω-MCA | 0 | 227 | 446 | 1149 | 0 | 0 | 0 | 0 |
| β-MCA | 0 | 66 | 167 | 453 | 0 | 0 | 0 | 0 |
| LCA | 0 | 2 | 8 | 7 | 0 | 0 | 0 | 0 |
| LCAS | 0 | 0 | 2 | 1 | 0 | 0 | 0 | 0 |
| GCA | 27 | 277 | 694 | 1393 | 27 | 36 | 38 | 45 |
| GMCA | 0 | 58 | 186 | 459 | 0 | 0 | 0 | 0 |
| GUDCA | 0 | 3 | 11 | 26 | 0 | 0 | 0 | 0 |
| GHDCA | 0 | 47 | 84 | 150 | 0 | 0 | 0 | 0 |
| GCDCA | 17 | 131 | 185 | 360 | 17 | 24 | 37 | 33 |
| GDCA | 0 | 29 | 77 | 138 | 0 | 0 | 0 | 0 |
| GLCA | 0 | 5 | 18 | 29 | 0 | 0 | 0 | 0 |
| TCA | 3 | 59 | 333 | 711 | 3 | 3 | 5 | 5 |
| TMCA | 0 | 20 | 25 | 73 | 0 | 0 | 0 | 0 |
| TUDCA | 0 | 3 | 32 | 56 | 0 | 0 | 0 | 0 |
| THDCA | 0 | 0 | 17 | 67 | 0 | 0 | 0 | 0 |
| TCDCA | 0 | 57 | 78 | 169 | 0 | 0 | 0 | 0 |
| TDCA | 0 | 31 | 172 | 373 | 0 | 0 | 0 | 0 |
| TLCA | 0 | 0 | 20 | 23 | 0 | 0 | 0 | 0 |

**Table 3: Refers to the data in Figure 2F**

| **bile acid [nmol/L]** | **rat bmMSC 2 µM TUDCA** | | | | **rat bmMSC control** | | | |
|---|---|---|---|---|---|---|---|---|
| | **1d** | **7d** | **14d** | **21d** | **1d** | **7d** | **14d** | **21d** |
| CA | 121 | 362 | 1082 | 2232 | 121 | 115 | 127 | 106 |
| CDCA | 0 | 170 | 585 | 1158 | 0 | 0 | 0 | 0 |
| DCA | 236 | 663 | 1155 | 2706 | 236 | 190 | 189 | 204 |
| UDCA | 0 | 35 | 63 | 129 | 0 | 0 | 0 | 0 |
| HDCA | 2 | 138 | 403 | 792 | 2 | 0 | 0 | 0 |
| α/ω-MCA | 0 | 181 | 361 | 919 | 0 | 0 | 0 | 0 |
| β-MCA | 0 | 55 | 128 | 368 | 0 | 0 | 0 | 0 |
| LCA | 0 | 3 | 14 | 10 | 0 | 0 | 0 | 0 |
| LCAS | 0 | 0 | 0 | 1 | 0 | 0 | 0 | 0 |
| GCA | 29 | 216 | 518 | 1116 | 29 | 24 | 36 | 31 |
| GMCA | 0 | 47 | 160 | 350 | 0 | 0 | 0 | 0 |
| GUDCA | 0 | 7 | 8 | 15 | 0 | 0 | 0 | 0 |
| GHDCA | 0 | 33 | 62 | 138 | 0 | 0 | 0 | 0 |
| GCDCA | 19 | 118 | 149 | 318 | 19 | 24 | 20 | 27 |
| GDCA | 0 | 25 | 48 | 103 | 0 | 0 | 0 | 0 |
| GLCA | 0 | 7 | 15 | 22 | 0 | 0 | 0 | 0 |
| TCA | 4 | 43 | 264 | 552 | 4 | 4 | 5 | 7 |
| TMCA | 0 | 18 | 26 | 54 | 0 | 0 | 0 | 0 |
| TUDCA | 0 | 6 | 21 | 52 | 0 | 0 | 0 | 0 |
| THDCA | 0 | 0 | 11 | 18 | 0 | 0 | 0 | 0 |
| TCDCA | 0 | 48 | 57 | 155 | 0 | 0 | 0 | 0 |
| TDCA | 0 | 34 | 128 | 360 | 0 | 0 | 0 | 0 |
| TLCA | 0 | 0 | 6 | 19 | 0 | 0 | 0 | 0 |

**Table 4: Refers to the data in Figure 6D**

| **bile acid [nmol/L]** | **wild type bmMSC 2 µM TUDCA** | | | | **FXR-KO bmMSC 2 µM TUDCA** | | | |
|---|---|---|---|---|---|---|---|---|
| | **1d** | **7d** | **14d** | **21d** | **1d** | **7d** | **14d** | **21d** |
| α/ω-MCA | 0 | 26 | 57 | 83 | 0 | 0 | 11 | 15 |
| β-MCA | 0 | 94 | 176 | 267 | 0 | 0 | 42 | 54 |
| CA | 0 | 190 | 440 | 854 | 0 | 15 | 69 | 95 |
| UDCA | 0 | 6 | 15 | 29 | 0 | 0 | 3 | 5 |
| HDCA | 0 | 89 | 192 | 279 | 0 | 0 | 25 | 33 |
| CDCA | 0 | 89 | 148 | 478 | 0 | 0 | 56 | 61 |
| DCA | 12 | 69 | 151 | 178 | 9 | 21 | 62 | 85 |
| TMCA | 0 | 32 | 62 | 76 | 0 | 0 | 14 | 27 |
| TCA | 0 | 95 | 168 | 192 | 0 | 39 | 72 | 81 |
| THDCA | 0 | 81 | 161 | 277 | 0 | 0 | 0 | 0 |
| TCDCA | 0 | 15 | 36 | 49 | 0 | 3 | 10 | 12 |
| TDCA | 0 | 6 | 9 | 13 | 0 | 0 | 0 | 0 |
| GMCA | 0 | 97 | 159 | 225 | 0 | 0 | 21 | 38 |
| GCA | 0 | 44 | 70 | 197 | 0 | 0 | 18 | 22 |
| GHDCA | 0 | 14 | 22 | 48 | 0 | 0 | 10 | 10 |
| GCDCA | 0 | 33 | 59 | 90 | 0 | 0 | 0 | 0 |
| GDCA | 0 | 6 | 9 | 17 | 0 | 0 | 2 | 3 |

**Table 5: Refers to the data in Figure 6H**

| **bile acid [nmol/L]** | **wild type bmMSC 2 µM TUDCA** | | | | **TGR5-KO bmMSC 2 µM TUDCA** | | | |
|---|---|---|---|---|---|---|---|---|
| | **1d** | **7d** | **14d** | **21d** | **1d** | **7d** | **14d** | **21d** |
| α/ω-MCA | 0 | 2118 | 2585 | 2816 | 0 | 331 | 412 | 463 |
| β-MCA | 0 | 2500 | 2156 | 2701 | 0 | 434 | 537 | 654 |
| CA | 275 | 13015 | 18493 | 19588 | 265 | 1676 | 2074 | 2419 |
| UDCA | 0 | 1003 | 1098 | 1163 | 0 | 191 | 230 | 245 |
| HDCA | 0 | 8411 | 8541 | 8997 | 0 | 1048 | 1508 | 1485 |
| CDCA | 0 | 14013 | 13390 | 16809 | 0 | 1515 | 2036 | 1890 |
| DCA | 1066 | 6788 | 6505 | 6984 | 2449 | 9773 | 9666 | 9574 |
| TMCA | 0 | 230 | 239 | 268 | 0 | 47 | 58 | 87 |
| TCA | 5 | 821 | 845 | 969 | 12 | 151 | 175 | 192 |
| THDCA | 0 | 0 | 0 | 0 | 0 | 0 | 0 | 0 |
| TCDCA | 10 | 1130 | 1257 | 1406 | 0 | 349 | 469 | 469 |
| TDCA | 4 | 391 | 625 | 703 | 0 | 0 | 156 | 180 |
| GMCA | 36 | 961 | 1132 | 1335 | 45 | 103 | 148 | 155 |
| GCA | 56 | 3123 | 4413 | 4900 | 45 | 426 | 452 | 561 |
| GHDCA | 0 | 481 | 521 | 574 | 0 | 107 | 127 | 134 |
| GCDCA | 310 | 1891 | 2532 | 2744 | 361 | 307 | 528 | 535 |
| GDCA | 29 | 381 | 468 | 454 | 0 | 40 | 47 | 27 |

**Table 6: Refers to the data in Figure 7E**

| **bile acid [nmol/L]** | **wild type bmMSC HGF/FGF4** | | | | **FXR-KO bmMSC HGF/FGF4** | | | |
|---|---|---|---|---|---|---|---|---|
| | **1d** | **7d** | **14d** | **21d** | **1d** | **7d** | **14d** | **21d** |
| α/ω-MCA | 0 | 28 | 53 | 74 | 0 | 28 | 47 | 72 |
| β-MCA | 0 | 96 | 151 | 243 | 0 | 98 | 178 | 254 |
| CA | 0 | 219 | 304 | 773 | 0 | 218 | 346 | 719 |
| UDCA | 0 | 6 | 15 | 26 | 0 | 6 | 13 | 18 |
| HDCA | 0 | 96 | 171 | 275 | 0 | 92 | 163 | 279 |
| CDCA | 0 | 69 | 153 | 475 | 0 | 73 | 154 | 421 |
| DCA | 12 | 83 | 155 | 171 | 9 | 72 | 111 | 139 |
| TMCA | 0 | 34 | 55 | 61 | 0 | 39 | 49 | 67 |
| TCA | 0 | 121 | 167 | 180 | 0 | 134 | 152 | 167 |
| THDCA | 0 | 79 | 163 | 260 | 0 | 78 | 145 | 203 |
| TCDCA | 0 | 12 | 33 | 46 | 0 | 14 | 31 | 43 |
| TDCA | 0 | 3 | 7 | 15 | 0 | 4 | 9 | 12 |
| GMCA | 0 | 82 | 124 | 184 | 0 | 67 | 96 | 196 |
| GCA | 0 | 40 | 65 | 113 | 0 | 33 | 45 | 89 |
| GHDCA | 0 | 15 | 26 | 46 | 0 | 14 | 23 | 44 |
| GCDCA | 0 | 33 | 56 | 68 | 0 | 31 | 41 | 61 |
| GDCA | 0 | 5 | 6 | 14 | 0 | 5 | 8 | 13 |

**Table 7: Refers to the data in Figure 7F**

| **bile acid [nmol/L]** | **wild type bmMSC HGF/FGF4** | | | | **TGR5-KO bmMSC HGF/FGF4** | | | |
|---|---|---|---|---|---|---|---|---|
| | **1d** | **7d** | **14d** | **21d** | **1d** | **7d** | **14d** | **21d** |
| α/ω-MCA | 0 | 1213 | 1338 | 1615 | 0 | 1493 | 2037 | 2904 |
| β-MCA | 0 | 1310 | 1638 | 1859 | 0 | 1941 | 2588 | 3544 |
| CA | 275 | 6213 | 13699 | 15728 | 265 | 7140 | 10037 | 16750 |
| UDCA | 0 | 429 | 559 | 660 | 0 | 452 | 804 | 1171 |
| HDCA | 0 | 4615 | 6796 | 7758 | 0 | 5181 | 8173 | 13806 |
| CDCA | 0 | 6528 | 7974 | 9694 | 0 | 8702 | 10990 | 17579 |
| DCA | 1066 | 4334 | 4783 | 6321 | 2449 | 10515 | 10408 | 10079 |
| TMCA | 0 | 134 | 140 | 211 | 0 | 181 | 274 | 414 |
| TCA | 5 | 443 | 763 | 761 | 12 | 536 | 699 | 1078 |
| THDCA | 0 | 631 | 643 | 553 | 0 | 800 | 950 | 1497 |
| TCDCA | 10 | 415 | 1497 | 1567 | 0 | 499 | 727 | 1010 |
| TDCA | 4 | 246 | 649 | 732 | 0 | 234 | 259 | 439 |
| GMCA | 36 | 452 | 974 | 1426 | 45 | 510 | 787 | 1213 |
| GCA | 56 | 1471 | 3710 | 4387 | 45 | 1690 | 2239 | 4013 |
| GHDCA | 0 | 294 | 354 | 487 | 0 | 401 | 481 | 608 |
| GCDCA | 310 | 1056 | 1748 | 2002 | 361 | 1176 | 1503 | 2292 |
| GDCA | 29 | 187 | 495 | 580 | 0 | 194 | 227 | 334 |

### Example 1: Bile acids induce hepatic differentiation in HSCs.

The finding that bile acids can support liver regeneration [7] prompted the inventors to investigate, whether bile acids can influence the hepatic differentiation of HSC and other MSC from the bone marrow or umbilical cord blood of rodents. It turned out, that various bile acids such as GCDCA (glycochenodeoxycholic acid), TUDCA (tauroursodeoxycholic acid), CA (cholic acid), GCDCA (glycochenodeoxycholic acid), TCA (taurocholic acid), TCDCA (taurochenodeoxycholic acid), and TLCA (taurolithocholic acid) can promote the differentiation of HSC into hepatocytes in vitro (Figure 1). Primary cultures of rat HSC were treated with 2 µM of individual bile acids dissolved in a serum-free medium for 21 days. The initiation of hepatic differentiation of HSC was indicated by the albumin protein production and release into the culture supernatant, which was measured by an enzyme-linked immunosorbent assay (ELISA) specific for rat albumin. Among the bile acids tested, TUDCA was found to be most potent (Figure 1). TUDCA was also tested at lower (0.5 µM and 1 µM) and higher (20 µM and 100 µM) concentrations on primary cultures of HSC. Compared to 2 µM TUDCA, the other concentrations of TUDCA led to lower albumin expression, indicating insufficient hepatic differentiation or adverse effects. Therefore, 2 µM TUDCA were used in the following experiments. TUDCA-mediated induction of hepatic differentiation was not only observed in liver-resident MSC (stellate cells), but also in MSC from the bone marrow of rats as indicated by the induction of albumin expression and bile acid production, which were determined by qPCR of albumin mRNA in adherent cells and ELISA for rat albumin as well as ultra-high-performance liquid chromatography-tandem mass spectrometry (UHPLC-MS/MS) of bile acids in culture supernatants (Figure 2). In contrast to this, MSC of the control, which received the same medium but without TUDCA, showed no hints of hepatic differentiation (Figure 2).

### Example 2: Bile acids induce hepatic differentiation in MSCs

Also clonally expanded HSC and umbilical cord blood stem cells with characteristics of MSC were able to differentiate into hepatocytes in vitro in response to TUDCA treatment (Figure 3). This effect was obviously limited to stem cells such as MSC, since muscle fibroblasts from the abdominal muscle of rats were unable to differentiate into hepatocytes within 21 days of TUDCA treatment (Figure 3).

### Example 3: TUDCA induces differentiation into hepatic progenitor cells and mature hepatocytes

During TUDCA-initiated hepatic differentiation MSC from the liver and bone marrow of rats lower the expression of mesodermal markers such as desmin and transiently acquire the expression profile of hepatic progenitor cells before they differentiate into hepatocytes as investigated by qPCR (Figure 4). Hepatic progenitor cells are characterized by the expression of keratin 19 (K19), epithelial cell adhesion molecule (EPCAM) and α-fetoprotein (AFP), whereas hepatocytes typically express albumin along with keratin 18, cytochrome P450 7A1 (CYP7A1) and hepatocyte nuclear factor 4α (HNF4α). The hepatocyte markers albumin, CYP7A1 and HNF4α steadily increased during TUDCA treatment, which indicated progressing differentiation of MSC into hepatocytes with time (Figure 2 and 4). The formation of hepatic progenitor cells and hepatocytes by MSC was confirmed by immunofluorescence staining (Figure 5). Cells with epithelial markers such as K18 and K19 as well as residual mesodermal filaments such as desmin and vimentin were found after 14 and 21 days of TUDCA treatment. More mature hepatocytes without mesodermal markers were also detected at day 21 of TUDCA treatment by immunofluorescence staining (Figure 5).

### Example 4: Bile acid receptors mediate bile acid dependent hepatic differentiation

The molecular mechanism that underlies TUDCA-mediated hepatic differentiation of MSC was investigated by the use of isolated bmMSC from farnesoid X receptor (FXR) and G protein-coupled bile acid receptor 1 (TGR5) knockout mice. A knockout of either FXR or TGR5 significantly inhibited the TUDCA-mediated hepatic differentiation of bmMSC as indicated by decreased albumin expression and bile acid production (Figure 6). This suggests that both bile acid receptors are involved in hepatic differentiation through bile acids. However, growth factor-initiated hepatic differentiation was found to be independent from bile acid receptors, since the differentiation of MSC into hepatocytes was found to be unaffected in bmMSC from FXR and TGR5 knockout mice during treatment with hepatocyte growth factor (HGF) and fibroblast growth factor 4 (FGF4) (Figure 7).

In conclusion, the treatment of MSC with bile acids or related compounds represents a novel technique to generate hepatocytes in vitro. MSC can be easily isolated from various tissues and expanded in culture, which ensures their availability for cost-efficient hepatocyte production via bile acid treatment. Affordable and simple techniques to generate patient-specific hepatocytes from stem cells are required for therapeutic or industrial use in the future.

### References

1. Kordes C, et al. Hepatic stellate cells support hematopoiesis and are liver-resident mesenchymal stem cells. Cell Physiol Biochem. 2013;31:290-304.
2. Kordes C, et al. Stellate cells from rat pancreas are stem cells and can contribute to liver regeneration. PLoS One 2012;7:e51878.
3. Sato Y, et al. Human mesenchymal stem cells xenografted directly to rat liver are differentiated into human hepatocytes without fusion. Blood 2005;106:756-763.
4. Chamberlain J, et al. Efficient generation of human hepatocytes by the intrahepatic delivery of clonal human mesenchymal stem cells in fetal sheep. Hepatology 2007;46;1935-1945.
5. Aurich I, et al. Functional integration of hepatocytes derived from human mesenchymal stem cells into mouse livers. Gut 2007;56:405-415.
6. Kordes C, et al. CD133+ hepatic stellate cells are progenitor cells. Biochem Biophys Res Commun. 2007;352:410-417.
7. Huang W, et al. Nuclear receptor-dependent bile acid signaling is required for normal liver regeneration. Science 2006;312:233-236.

## Claims

1. A method of promoting hepatic differentiation of a stem cell, comprising contacting the stem cell with an effective amount of a bile acid receptor agonist.

2. A method for producing a hepatocyte or a hepatocyte progenitor cell from a stem cell via promoting hepatic differentiation in the stem cell, comprising providing a stem cell and contacting the stem cell with an effective amount of a bile acid receptor agonist.

3. The method according to claim 1 or 2, which is an *ex-vivo* or *in-vitro* method.

4. The method according to any of claims 1 to 3, wherein the stem cell is a mesenchymal stem cell (MSC), such as a liver resident MSC (hepatic stellate cell (HSC)), or a bone marrow MSC.

5. The method according to any of claims 1 to 4, wherein said bile acid receptor agonist is a bile acid selected from the group comprising tauroursodeoxycholic acid (TUDCA), cholic acid (CA), glycochenodeoxycholic acid (GCDCA), taurocholic acid (TCA), taurochenodeoxycholic acid (TCDCA), taurolithocholic acid (TLCA) and derivatives or analogs of these compounds.

6. The method according to any of claims 1 to 5, wherein hepatic differentiation is **characterized by** an increased expression of at least one hepatocyte differentiation marker upon contacting the stem cells with the bile acid receptor agonist.

7. The method according to any of claims 1 to 6, comprising the further step of testing the expression of at least one hepatocyte differentiation marker.

8. A bile acid receptor agonist for use in the treatment of a patient suffering from a liver disease, wherein the treatment is a cell, tissue or organ transplantation and comprises the steps of a method of any of claims 1 to 7, and administering said hepatocyte or hepatocyte progenitor cell to the patient.

9. The bile acid receptor agonist according to claim 8, wherein said stem cell is an autologous stem cell from said patient, and optionally comprises as first step obtaining the autologous stem cell from the patient.

10. The bile acid receptor agonist according to claim 8 or 9, wherein said administration is a cell transplantation, for example a transplantation of the hepatocyte or hepatocyte progenitor cell into the liver of the patient.

11. The bile acid receptor agonist according to any of claims 8 to 10, wherein said liver disease is hepatitis, cirrhosis, acute liver failure, acute liver infections, acute chemical toxicity, chronic liver failure, cholangiocitis, biliary cirrhosis, Alagille syndrome, alpha-1-antitrypsin deficiency, autoimmune hepatitis, biliary atresia, cancer of the liver, cystic disease of the liver, fatty liver, galactosemia, gallstones, Gilbert's syndrome, hemochromatosis, hepatitis A, hepatitis B, hepatitis C and other hepatitis viral infections, poryphyria, primary sclerosing cholangitis, Reye's syndrome, sarcoidosis, tyrosinemia, type 1 glycogen storage disease or Wilson's disease.

12. The bile acid receptor agonist according to claim 11, wherein the treatment comprises the administration of the bile acid receptor agonist to the patient.

13. Use of a bile acid receptor agonist for inducing hepatocyte differentiation of a stem cell.

14. The use according to claim 13 which is an *in-vitro* or *ex-vivo* use.

15. A kit adapted for the performance of a method according to any of claims 1 to 7, the kit comprising a bile acid receptor agonist.
